(19) Europäisches Patentamt
European Patent Office
Office européen des brevets

(11) Veröffentlichungsnummer: **0 498 418 B1**

(12) **EUROPÄISCHE PATENTSCHRIFT**

(45) Veröffentlichungstag der Patentschrift: **18.01.95**

(51) Int. Cl.$^{6}$: **C07D 307/33**

(21) Anmeldenummer: **92101979.0**

(22) Anmeldetag: **06.02.92**

# (54) Verfahren zur Herstellung von Homoserinlactonen.

(30) Priorität: **08.02.91 DE 4103821**

(43) Veröffentlichungstag der Anmeldung:
**12.08.92 Patentblatt 92/33**

(45) Bekanntmachung des Hinweises auf die Patenterteilung:
**18.01.95 Patentblatt 95/03**

(84) Benannte Vertragsstaaten:
**AT BE CH DE DK ES FR GB IT LI NL SE**

(56) Entgegenhaltungen:

**TETRAHEDRON LETTERS. Bd. 25, Nr. 9, 1984, OXFORD GB Seiten 927 - 930; B.H. LEEET AL.: 'CONSTITUENTS OF MICROBIAL IRON CHELATORS. THE SYNTHESIS OF OPTICALLYACTIVES OF DERIVATIVES OF delta-N-HYDROXY-L-ORNITHINE.'**

**TETRAHEDRON, (INCL. TETRAHEDRON REPORTS) Bd. 42, Nr. 23, 1986, OXFORD GB Seiten6551 - 6554; J.E. BALDWIN ET AL.: 'SYNTHESIS OF BICYCLIC gamma-LACTAMS VIAOXAZOLIDINONES'**

**HELVETICA CHIMICA ACTA. Bd. 73, 1990, BASEL CH Seiten 405 - 410; G. BOLD ET AL. : '43. Herstellung von 'Semialdehyd'-Derivaten von Asparaginsäure- undGlutaminsäure durch Rosenmund-Reduktion'**

(73) Patentinhaber: **Hoechst Schering AgrEvo GmbH**
**Gerichtstrasse 27**
**D-13342 Berlin (DE)**

(72) Erfinder: **Hoffmann, Michael, Dr.**
**Delkenheimer Strasse 5**
**W-6238 Hofheim am Taunus (DE)**
Erfinder: **Zeiss, Hans-Joachim, Dr.**
**Hauptstrasse 127**
**W-6231 Sulzbach/Taunus (DE)**

**EP 0 498 418 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur enantioselektiven Synthese von Homoserinlactonen der Formel I,

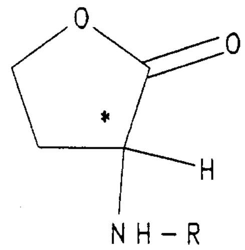

( I )

worin

R Wasserstoff, Alkyl, Alkenyl, Alkinyl, wobei die vorstehenden drei Reste Heteroatome in der Kette besitzen können, Alkylcarbonyl, Alkoxycarbonyl, Alkylsulfonyl, Benzyl, Benzyloxycarbonyl, Aryloxycarbonyl oder Arylsulfonyl, wobei die vier letztgenannten Reste im Arylteil gegebenenfalls substituiert sein können,

bedeutet, sowie deren Salze mit anorganischen oder organischen Säuren oder Basen.

α-Amino-γ-butyrolactone (Homoserinlactone) sind bei vielen biologisch wirksamen Verbindungen ein zentrales Strukturelement. So kommen sie zum Beispiel als wichtige Bausteine bei Renininhibitoren (WO 8705909), ACE-Inhibitoren (EP-A-266568) und bei Fungiziden (EP-A-51742 oder DE-A-2804299) vor. Sie können auch als Sedativa (EP-A-151964) und zur Behandlung von Alkoholismus (EP-A-144812) eingesetzt werden. Außerdem stellen optisch aktive Homoserinlactone wertwolle Synthone zur Darstellung enantiomerenreiner Derivate dar. So können sie zum Beispiel auch in die entsprechenden Homoserinderivate überführt werden, welche dann ihrerseits wieder wichtige Strukturelemente von biologisch wirksamen Verbindungen sind (Tetrahedron Lett. 1978 (26) 2243-2246).

Die bislang bekannten Synthesen zur Herstellung von optisch aktiven Homoserinlactonen sind mit Nachteilen behaftet.

So haben die Verfahren, welche von optisch aktiven Methioninderivaten ausgehen, den Nachteil, daß D- bzw. L-Methionin relativ teuer oder schlecht zugänglich sind. Hinzu kommt der hohe Preis und die extrem hohe Toxizität von Jodmethan, welches bei diesen Verfahren eingesetzt wird (JP-48076857 (Derwent V 4816 (1974)), JP-48018229 (Derwent V 14063 (1974)), Bull. Chem. Soc. 46, 669 (1973)).

Weiterhin sind in der Literatur Verfahren beschrieben, welche von D-bzw. L-Asparaginsäurederivaten ausgehend über eine Reduktion mit Metallhydriden zu optisch aktiven Homoserinlactonen führen (Tetrahedron 1988, 637; J. Org. Chem. 1990, 55, 4763). Diese Verfahren sind aufgrund der hohen Preise der einzusetzenden Komplexen Metallhydride und deren problematischen Abfallbeseitigung wirtschaftlich gesehen nicht sinnvoll.

Gefordert und von erheblichem Vorteil ist daher ein Verfahren, welches die Synthese von Homoserinlactonen aus preiswerten und leicht zugänglichen Ausgangsstoffen ermöglicht.

Gegenstand der vorliegenden Erfindung ist ein Verfahren zur Herstellung von racemischen oder optisch aktiven Homoserinlactonen der genannten allgemeinen Formel I, dadurch gekennzeichnet, daß man racemische bzw. optisch aktive Aldehyde der allgemeinen Formel II,

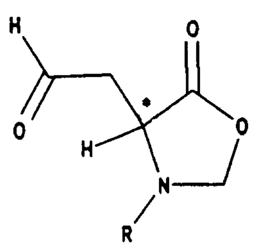

( II )

worin R die gleiche Bedeutung wie in Formel I besitzt, in Gegenwart eines für die Hydrierung geeigneten Katalysators hydriert.

Das erfindungsgemäße Verfahren stellt eine katalytische Hydrierung der Aldehydgruppe zum Alkohol und unter Öffnung des Oxazolidinrings und Abspaltung von Formaldehyd eine Lactonisierung dar.

In den Formeln sind Alkyl, Alkenyl und Alkinyl geradkettig oder verzweigt. Entsprechendes gilt für die Alkylreste in den zusammengesetzten Gruppen wie Alkylcarbonyl, Alkoxycarbonyl, Alkylsulfonyl etc.; Alkyl bedeutet z.B. Methyl, Ethyl, n- und i-Propyl, n-, i-, t- und 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl bedeutet z.B. Allyl, 1-Methylprop-2-en-1-yl, But-2-en-1-yl, But-3-en-1-yl,
1-Methyl-but-3-enyl und 1-Methyl-but-2-enyl; Alkinyl bedeutet z.B. Propargyl, But-2-in-1-yl, But-3-in-1-yl, 1-Methyl-but-3-inyl; Aryl bedeutet z.B. Phenyl, Naphthyl, Thienyl u.a. Heteroaryle; gegebenenfalls substituiertes Aryl ist z.B. Aryl, das unsubstituiert oder ein- oder mehrfach durch Reste aus der Gruppe Halogen, Alkyl, Alkoxy, Halogenalkyl, Halogenalkoxy und Nitro substituiert ist.

In der Formel I bedeutet R vorzugsweise
Wasserstoff, $C_1$-$C_6$-Alkyl, $C_2$-$C_6$-Alkenyl, $C_2$-$C_6$-Alkinyl, wobei die drei letztgenannten Reste in der Kette Heteroatome, beispielsweise in Form ein oder mehrerer divalenter Gruppen wie -O-, -S-, -NH- und -N($CH_3$)-, enthalten können, oder ($C_1$-$C_6$-Alkyl)-carbonyl, ($C_1$-$C_6$-Alkoxy)-carbonyl, Benzyl, Benzyloxycarbonyl, Phenyloxycarbonyl oder Phenylsulfonyl, wobei die vier letztgenannten Reste im Phenylteil unsubstituiert oder durch einen oder mehrere Reste aus der Gruppe Halogen, $C_1$-$C_4$-Alkyl, $C_1$-$C_4$-Alkoxy, $C_1$-$C_4$-Halogenalkyl, $C_1$-$C_4$-Halogenalkoxy und Nitro substituiert sind.

R ist insbesondere
Wasserstoff, $C_1$-$C_4$-Alkyl, $C_2$-$C_4$-Alkenyl, $C_2$-$C_4$-Alkinyl, ($C_1$-$C_4$-Alkyl)-carbonyl, ($C_1$-$C_4$-Alkoxy)-carbonyl, Benzyl, Benzyloxycarbonyl, Phenyloxycarbonyl, Phenylsulfonyl, wobei die vier letztgenannten Reste unsubstituiert oder durch ein oder mehrere Reste aus der Gruppe Methyl, Ethyl, Methoxy, Ethoxy und Nitro substituiert sind.

Die Ausgangsstoffe der Formel II sind nach literaturbekannten Methoden, z.B. über eine Rosenmund-Reduktion aus den entsprechenden racemischen bzw. optisch aktiven Carbonsäurechloriden der Formel III,

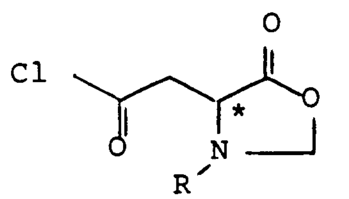

III

worin R die gleiche Bedeutung hat wie in Formel I, bequem zugänglich (Helvetica Chim. Acta 73, 405-(1990); Tetrahedron 42 (1986) 6551).

Die Säurechloride der Formel III sind zum Teil beschrieben oder können nach literaturbekannten Methoden aus den entsprechenden Carbonsäuren der Formel IV,

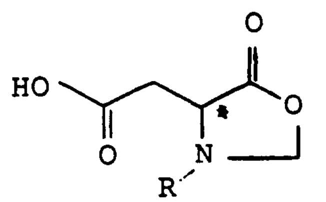

IV

worin R die gleiche Bedeutung wie in Formel I besitzt, hergestellt werden (Organikum, VEB Deutscher Verlag der Wissenschaften, Berlin 1976, S. 526; Helvetica Chim. Acta 73 (1990) 405).

Die Carbonsäuren der Formel IV sind zum Teil literaturbekannt oder können nach an sich bekannten Verfahren aus (S)- bzw. (R)-Asparaginsäurederivaten bzw. deren Gemischen der Formel V,

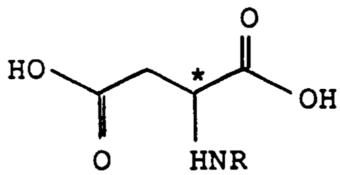

V

worin R die gleiche Bedeutung wie in Formel I hat, hergestellt werden (Synthesis 1989, 542; Chem. Ber. 95, 1009 (1962); Tetrahedron 42 (1986) 6551).

Hydrierungen von Aldehyden, die sich strukturell von denen der Formel II unterscheiden, in Gegenwart von Katalysatoren sind bekannt (Chem. Commun. 1967, 923; Chemistry Lett. 1988, 1695; Chemistry Lett.1977, 1085; Synthesis 1988, 966).

Überraschend an dem erfindungsgemäßen Verfahren ist aber beispielsweise, daß man die Reduktion der Aldehyde der Formel II, welche eine $\alpha$-Aminosäuregruppierung erhalten, auch in Gegenwart der unterschiedlichsten Reste R chemoselektiv durchführen kann. Weiterhin überraschend ist auch, daß der Alkohol unter Öffnung des Oxazolidinringes zu den Homoserinlactonen der Formel I umlagert und daß bei dieser Reaktion keine Racemisierung stattfindet.

Das erfindungsgemäße Verfahren ermöglicht somit insbesondere die Herstellung optisch aktiver Homoserinlactone aus einfach zugänglichen optisch aktiven Asparaginsäurederivaten in enantioselektiver Weise. Bevorzugt werden optisch aktive Ausgangsstoffe eingesetzt und optisch aktive Verbindungen der Formel (I) erhalten, die eine optische Reinheit von mehr als 50 %, vorzugsweise mehr als 80 %, insbesondere mehr als 30 % (S)-Form bzw. (R)-Form enthalten.

Das erfindungsgemäße Verfahren kann beispielsweise so durchgeführt werden, daß man die Aldehyde der Formel II in einem geeigneten anorganischen oder organischen Lösungsmittel mit einem geeigneten Katalysator versetzt und die Hydrierung bei Temperaturen von 30 bis 200 °C, bevorzugt bei 50 bis 150 °C, und bei einem Wasserstoff-Druck von 1 bar - 100 bar, bevorzugt 1 bar - 20 bar, durchführt.

Als Lösungsmittel eignen sich organische Lösungsmittel aus der Gruppe der Alkohole, wie z.B. Methanol, Ethanol, n- und i-Propanol, n- und i-Butanol, aus der Gruppe der aliphatischen und aromatischen Kohlenwasserstoffe, wie z.B. Cyclohexan und Petroläther bzw. Benzol, Toluol und Xylole, aus der Gruppe der Ether, wie z.B. (Poly)-glykol-monoalkylether oder -dialkylether, Diethylether, Tetrahydrofuran und Dioxan, sowie aus der Gruppe der Ester, z.B. Essigester. Bevorzugt sind organische Lösungsmittel wie Benzol, Toluol und Tetrahydrofuran. Auch Gemische von organischen Lösungsmitteln können eingesetzt werden.

Als Katalysatoren eignen sich Hydrierungskatalysatoren, die unter den Reaktionsbedingungen in der Lage sind, die Reduktion einer Aldehydfunktion zu einer Hydroxymethylgruppe durch Wasserstoff zu katalysieren.

Beispielsweise eignen sich Katalysatoren aus der Gruppe Palladium, Ruthenium, Iridium und Platin sowie deren Komplexe bzw. Komplexsalze mit anorganischen und organischen Liganden. Besonders geeignet sind die zweiwertigen Salze von Ruthenium und die dreiwertigen Salze von Iridium, wobei ihre Triphenylphosphinkomplexe bevorzugt eingesetzt werden. Beispiele dafür sind z.B. $RuCl_2(PPh_3)_3$ und $IrH_3(PPh_3)_3$.

Das Gewichtsverhältnis von Substrat zu Katalysator kann in weitem Bereich variieren und hängt z.B. vom einzelnen Katalysator und der Umsatzrate ab. Ein hinsichtlich gewünschter Umsatzrate und Aufwand an Katalysatormenge optimales Verhältnis läßt sich in Vorversuchen leicht ermitteln. In der Regel sind gute Umsatzraten bei einem Gewichtsverhältnis Katalysator: Substrat von 1:10000 bis 1:20 möglich. Bei einem Katalysator-Komplex wie $RuCl_2(PPh_3)_3$ ist z.B. ein Gewichtsverhältnis von Katalysator:Substrat 1:500 bis 1:50 bevorzugt.

Die Isolierung des Produkts kann nach üblichen Methoden erfolgen. In der Regel destilliert man das Lösungsmittel unter reduziertem Druck ab. Das Rohprodukt, das in der Regel fest oder ein Öl ist, wird dann beispielsweise (um)kristallisiert oder chromatographiert.

Beispiel 1

N-(Benzyloxycarbonyl)-(S)-homoserinlacton

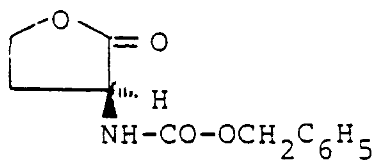

In einem 500 ml Stahlautoklaven (4 VA) legt man nacheinander 180 ml Benzol, 15,5 g (58,9 mmol) (S)-4-(Formylmethyl)-5-oxo-1,3-oxazolidin-3-carbonsäure-benzylester und 0,27 g $RuCl_2(PPh_3)_3$ vor, gibt einen Wasserstoffdruck von 10 bar auf und rührt 20 h bei 80 °C. Danach filtriert man ab und engt am Wasserstrahlvakuum ein. Das so erhaltene Rohprodukt wird aus Heptan/Essigsäureethylester umkristallisiert; man erhält 6,8 g (51 % d.Th.) des anfangs bezeichneten Produkts als farblose Kristalle, Schmp.: 124 - 126 °C $[\alpha]_D^{25}$ = -30,8 ° (c=1, MeOH); Literaturdaten (Bull. Chem. Soc. 46, 669 (1973)): Schmp.: 126 °C; $[\alpha]_D^{25}$ = -30,5 ° (c=1, MeOH).

Beispiel 2

N-(p-Toluolsulfonyl)-(S)-homoserinlacton

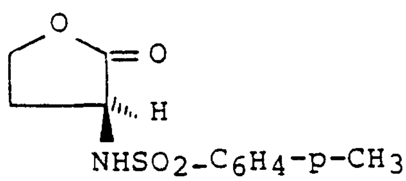

In einem 250 ml Stahlautoklaven (4 VA) legt man nacheinander 100 ml Benzol, 9,6 g (33,9 mmol) (S)-4-(Formylmethyl)-3-(p-toluolsulfonyl)-5-oxo-1,3-oxazolidin und 0,17 g $RuCl_2(PPh_3)_3$ vor, gibt einen Wasserstoffdruck von 10 bar auf und rührt 20 h bei 80 °C. Danach filtriert man ab und engt am Wasserstrahlvakuum ein. Das so erhaltene Rohprodukt wird aus Heptan/Essigsäureethylester umkristallisiert; man erhält 6 g (70 % d.Th) N-(p-Toluolsulfonyl)-(S)-homoserinlacton als farblose Kristalle. Schmp.: 131 - 133 °C; $[\alpha]_D^{25}$ = +8,5 ° (c=1, MeOH); Vergleichsdaten aus der Literatur (Bull. Chem. Soc. 46, 669 (1973)): Schmp.: 130 - 133 °C, $[\alpha]_D^{25}$ = +8,0° (c=1, MeOH).

Beispiel 3

N-Methoxycarbonyl-(S)-homoserinlacton

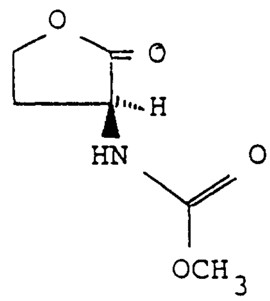

In einem 1 L-Stahlautoklaven (4 VA) legt man nacheinander 440 ml Benzol, 28 g (150 mmol) (S)-4-(Formylmethyl)-5-oxo-1,3-oxazolidin-3-carbonsäure-methylester und 0,7 g $RuCl_2(PPh_3)_3$ vor, gibt einen Wasserstoffdruck von 10 bar auf und rührt 20 h bei 80 °C. Danach filtriert man ab und engt am Wasserstrahlvakuum ein. Das Rohprodukt wird säulenchromatographisch an Kieselgel gereinigt (Laufmittel: Heptan/Essigsäureethylester = 3/7); man erhält 17,9 g (75 % d.Th.) Produkt als farblose Kristalle. Schmp.: 83 - 85 °C; $[\alpha]_D^{25}$ = -6,5 ° (c = 1, $CH_2Cl_2$); Literaturdaten (Tetrahedron Lett. 30 (1990) 2037); $[\alpha]_D^{25}$ = -6,8 ° (c = 1, $CH_2Cl_2$).

## Patentansprüche

**1.** Verfahren zur Herstellung von racemischen oder optisch aktiven Homoserinlactonen der allgemeinen Formel I,

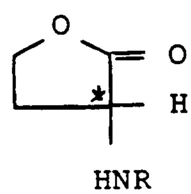

(I)

worin

R Wasserstoff, Alkyl, Alkenyl, Alkinyl, wobei die vorstehenden drei Reste Heteroatome in der Kette besitzen können, Alkylcarbonyl, Alkoxycarbonyl, Alkylsulfonyl, Benzyl, Benzyloxycarbonyl, Aryloxycarbonyl oder Arylsulfonyl, wobei die vier letztgenannten Reste im Arylteil gegebenenfalls substituiert sein können,

bedeutet, sowie deren Salze mit anorganischen oder organischen Säuren oder Basen, dadurch gekennzeichnet, daß man racemische bzw. optisch aktive Aldehyde der allgemeinen Formel II,

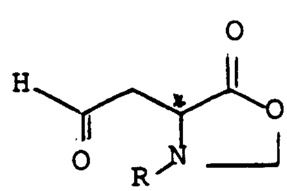

(II)

worin R die gleiche Bedeutung wie in Formel I besitzt, in Gegenwart eines für die Hydrierung geeigneten Katalysators hydriert.

2. Verfahren nach Anspruch 1, dadurch gekennzeichnet, daß R Wasserstoff, $C_1$-$C_6$-Alkyl, $C_2$-$C_6$-Alkenyl, $C_2$-$C_6$-Alkinyl, wobei die drei letztgenannten Reste in der Kette Heteroatome enthalten können, ($C_1$-$C_6$-Alkyl)-carbonyl, ($C_1$-$C_6$-Alkoxy)-carbonyl, Benzyl, Benzyloxycarbonyl, Phenyloxycarbonyl oder Phenylsulfonyl, wobei die vier letztgenannten Reste im Phenylteil unsubstituiert oder durch eine mehrere Reste aus der Gruppe Halogen, $C_1$-$C_4$-Alkyl, $C_1$-$C_4$-Alkoxy, $C_1$-$C_4$-Halogenalkyl, $C_1$-$C_4$-Halogenalkoxy und Nitro substituiert sind, bedeutet.

3. Verfahren nach Anspruch 1 oder 2, dadurch gekennzeichnet, daß R Wasserstoff, $C_1$-$C_4$-Alkyl, $C_2$-$C_4$-Alkenyl, $C_2$-$C_4$-Alkinyl, ($C_1$-$C_4$-Alkyl)-carbonyl, ($C_1$-$C_4$-Alkoxy)-carbonyl, Benzyl, Benzyloxycarbonyl, Phenyloxycarbonyl, Phenylsulfonyl, wobei die vier letztgenannten Reste unsubstituiert oder durch einen oder mehrere Reste aus der Gruppe Methyl, Ethyl, Methoxy, Ethoxy und Nitro substituiert sind, bedeutet.

4. Verfahren nach einem oder mehreren der Ansprüche 1 bis 3, dadurch gekennzeichnet, daß die Verbindungen der Formeln I (S)-Homoserinlactone oder (R)-Homoserinlactone mit einer optischen Reinheit von mehr als 50 % (S)- bzw. (R)-Form sind.

5. Verfahren nach einem oder mehreren der Ansprüche 1 bis 4, dadurch gekennzeichnet, daß die Hydrierung in Gegenwart eines organischen Lösungsmittels durchgeführt wird.

6. Verfahren nach Anspruch 5, dadurch gekennzeichnet, daß das Lösungsmittel aus der Gruppe Alkohole, aliphatische und aromatische Kohlenwasserstoffe, Ether und Ester und deren Gemische ausgewählt ist.

7. Verfahren nach Anspruch 6, dadurch gekennzeichnet, daß das Lösungsmittel Benzol, Toluol, ein Xylol oder Tetrahydrofuran ist.

8. Verfahren nach einem oder mehreren der Ansprüche 1 bis 7, dadurch gekennzeichnet, daß die Hydrierung bei 30 bis 200 °C durchgeführt wird.

9. Verfahren nach einem oder mehreren der Ansprüche 1 bis 8, dadurch gekennzeichnet, daß die Temperatur bei 50 bis 150 °C ist.

10. Verfahren nach einem oder mehreren der Ansprüche 1 bis 9, dadurch gekennzeichnet, daß der Wasserstoffdruck bei 1 bis 100 bar ist.

11. Verfahren nach einem oder mehreren der Ansprüche 1 bis 10, dadurch gekennzeichnet, daß der Wasserstoffdruck 1 bis 20 bar beträgt.

12. Verfahren nach einem oder mehreren der Ansprüche 1 bis 11, dadurch gekennzeichnet, daß der Katalysator aus der Gruppe Palladium, Ruthenium, Iridium und Platin sowie deren Komplexe und Komplexsalze mit anorganischen oder organischen Liganden ist.

13. Verfahren nach einem oder mehreren der Ansprüche 1 bis 12, dadurch gekennzeichnet, daß der Katalysator ein zweiwertiges Salz von Ruthenium oder ein dreiwertiges Salz von Iridium ist.

14. Verfahren nach einem oder mehreren der Ansprüche 1 bis 13, dadurch gekennzeichnet, daß der Katalysator $RuCl_2(PPh_3)_3$ ist.

15. Verfahren nach einem oder mehreren der Ansprüche 1 bis 14, dadurch gekennzeichnet, daß die Verbindungen der genannten Formel II aus Asparaginsäurederivaten der Formel V,

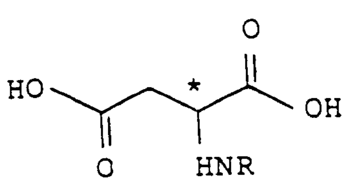

V

worin R wie in Formel (I) definiert ist, über Oxazolidinringbildung, anschließende Säurechloridbildung und Reduktion zum Aldehyd hergestellt werden.

**16.** Verwendung der Verbindungen der Formel II,

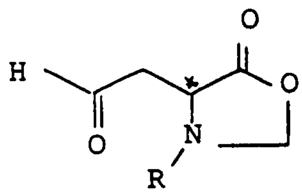

II

worin R die Bedeutung wie in Anspruch 1, 2 oder 3 hat, zur Herstellung von Homoserinlactonen.

## Claims

**1.** A process for the preparation of racemic or optically active homoserine lactones of the formula I

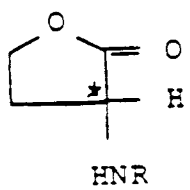

(I)

in which

R is hydrogen, alkyl, alkenyl, alkynyl, it being possible for the aforementioned three radicals to have hetero atoms in the chain, alkylcarbonyl, alkoxycarbonyl, alkylsulfonyl, benzyl, benzyloxycarbonyl, aryloxycarbonyl or arylsulfonyl, it being possible for the four last-mentioned radicals to be optionally substituted in the aryl moiety,

and the salts thereof with inorganic or organic acids or bases, which comprises racemic or optically active aldehydes of the formula II

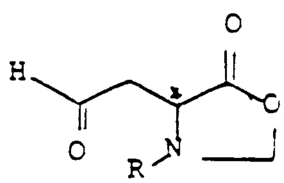

II

in which R has the same meaning as in formula I, being hydrogenated in the presence of a catalyst suitable for the hydrogenation.

**2.** The process as claimed in claim 1, wherein R is hydrogen, $C_1$-$C_6$-alkyl, $C_2$-$C_6$-alkenyl, $C_2$-$C_6$-alkynyl, it being possible for the three last-mentioned radicals to contain hetero atoms in the chain, or ($C_1$-$C_6$-alkyl)-carbonyl, ($C_1$-$C_6$-alkoxy)-carbonyl, benzyl, benzyloxycarbonyl, phenyloxycarbonyl or phenylsulfonyl, where the four last-mentioned radicals are unsubstituted or substituted by one or more radicals from the group comprising halogen, $C_1$-$C_4$-alkyl, $C_1$-$C_4$-alkoxy, $C_1$-$C_4$-haloalkyl, $C_1$-$C_4$-haloalkoxy and nitro in the phenyl moiety.

**3.** The process as claimed in claim 1 or 2, wherein R is hydrogen, $C_1$-$C_4$-alkyl, $C_2$-$C_4$-alkenyl, $C_2$-$C_4$-alkynyl, ($C_1$-$C_4$-alkyl)-carbonyl, ($C_1$-$C_4$-alkoxy)-carbonyl, benzyl, benzyloxycarbonyl, phenyloxycarbonyl, phenylsulfonyl, where the four last-mentioned radicals are unsubstituted or substituted by one or more radicals from the group comprising methyl, ethyl, methoxy, ethoxy and nitro.

**4.** The process as claimed in one or more of claims 1 to 3, wherein the compounds of the formula I are (S)-homoserine lactones or (R)-homoserine lactones with an optical purity of more than 50% (S) or (R) form.

**5.** The process as claimed in one or more of claims 1 to 4, wherein the hydrogenation is carried out in the presence of an organic solvent.

**6.** The process as claimed in claim 5, wherein the solvent is selected from the group comprising alcohols, aliphatic and aromatic hydrocarbons, ethers and esters and mixtures thereof.

**7.** The process as claimed in claim 6, wherein the solvent is benzene, toluene, xylene or tetrahydrofuran.

**8.** The process as claimed in one or more of claims 1 to 7, wherein the hydrogenation is carried out at 30 to 100 °C.

**9.** The process as claimed in one or more of claims 1 to 8, wherein the temperature is 50 to 150 °C.

**10.** The process as claimed in one or more of claims 1 to 9, wherein the hydrogen pressure is 1 to 100 bar.

**11.** The process as claimed in one or more of claims 1 to 10, wherein the hydrogen pressure is 1 to 20 bar.

**12.** The process as claimed in one or more of claims 1 to 11, wherein the catalyst is from the group comprising palladium, ruthenium, iridium and platinum and their complexes and complex salts with inorganic or organic ligands.

**13.** The process as claimed in one or more of claims 1 to 12, wherein the catalyst is a divalent salt of ruthenium or a trivalent salt of iridium.

**14.** The process as claimed in one or more of claims 1 to 13, wherein the catalyst is $RuCl_2(PPh_3)_3$.

**15.** The process as claimed in one or more of claims 1 to 14, wherein the compounds of the said formula II are prepared from aspartic acid derivatives of the formula V

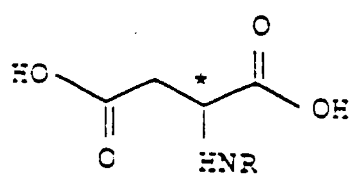

V

in which R is defined as in formula (I), via formation of an oxazolidine ring, subsequent formation of an acid chloride and reduction to the aldehyde.

**16.** The use of the compounds of the formula II

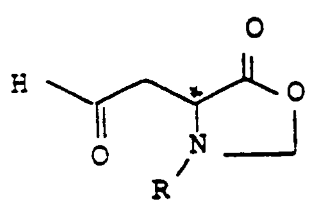

II

in which R has the meaning as in claim 1, 2 or 3 for the preparation of homoserine lactones.

## Revendications

**1.** Procédé pour la préparation de lactones d'homosérine racémiques ou optiquement actives, de formule générale I :

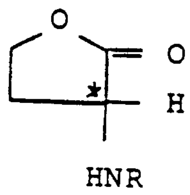

(I)

dans laquelle
R représente un atome d'hydrogène, un groupe alkyle, alcényle, alcynyle, les trois radicaux ci-dessus pouvant posséder des hétéroatomes dans la chaîne, alkylcarbonyle, alcoxycarbonyle, alkylsulfonyle, benzyle, benzyloxycarbonyle, aryloxycarbonyle ou arylsulfonyle, les quatre radicaux mentionnés en dernier pouvant éventuellement être substitués dans la partie aryle, ainsi que de leurs sels avec des acides ou des bases non-organiques ou organiques,
caractérisé en ce qu'on hydrogène des aldéhydes racémiques ou respectivement optiquement actifs de formule générale II :

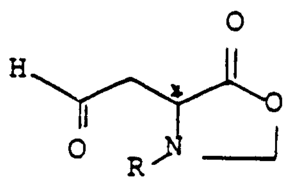

(II)

dans laquelle R possède la même signification que dans la formule I, en présence d'un catalyseur approprié pour l'hydrogénation.

**2.** Procédé selon la revendication 1, caractérisé en ce que R représente un atome d'hydrogène, un groupe alkyle en $C_1$-$C_6$, alcényle en $C_2$-$C_6$, alcynyle en $C_2$-$C_6$, les trois radicaux mentionnés en dernier pouvant contenir des hétéroatomes dans la chaîne, (alkyl en $C_1$-$C_6$)-carbonyle, (alcoxy en $C_1$-$C_6$)-carbonyle, benzyle, benzyloxycarbonyle, phényloxycarbonyle ou phénylsulfonyle, les quatre radicaux mentionnés en dernier étant, dans la partie phényle non-substitués ou substitués par un ou plusieurs radicaux choisis pris dans le groupe des halogènes, des groupes alkyle en $C_1$-$C_4$, alcoxy en $C_1$-$C_4$, halogénoalkyle en $C_1$-$C_4$, halogénoalcoxy en $C_1$-$C_4$ et nitro.

**3.** Procédé selon la revendication 1 ou 2, caractérisé en ce que R représente un atome d'hydrogène, un groupe alkyle en $C_1$-$C_4$, alcényle en $C_2$-$C_4$, alcynyle en $C_2$-$C_4$, (alkyl en $C_1$-$C_4$)-carbonyle, (alcoxy en $C_1$-$c_4$)-carbonyle, benzyle, benzyloxycarbonyle, phényloxycarbonyle, phénylsulfonyle, les quatre radicaux mentionnés en dernier étant, dans la partie phényle, non-substitués ou substitués par un ou plusieurs radicaux choisis pris dans le groupe constitué par méthyle, éthyle, méthoxy, éthoxy et nitro.

**4.** Procédé selon l'une ou plusieurs des revendications 1 à 3, caractérisé en ce que les composés de formule (I) sont des lactones de la (S)-homosérine ou des lactones de la (R)-homosérine ayant une pureté optique supérieure à 50 % de forme (S) ou respectivement (R).

**5.** Procédé selon l'une ou plusieurs des revendications 1 à 4, caractérisé en ce que l'hydrogénation est effectuée en présence d'un solvant organique.

**6.** Procédé selon la revendication 5, caractérisé en ce que le solvant est choisi dans le groupe des alcools, des hydrocarbures aliphatiques et aromatiques, des éthers et des esters, et de leurs mélanges.

**7.** Procédé selon la revendication 6, caractérisé en ce que le solvant est le benzène, le toluène, un xylène ou le tétrahydrofuranne.

**8.** Procédé selon l'une ou plusieurs des revendications 1 à 7, caractérisé en ce que l'hydrogénation est effectuée à une température de 30 à 200 ° C.

**9.** Procédé selon l'une ou plusieurs des revendications 1 à 8, caractérisé en ce que la température est de 50 à 150 ° C.

**10.** Procédé selon l'une ou plusieurs des revendications 1 à 9, caractérisé en ce que la pression d'hydrogène est de 1 à 100 bar.

**11.** Procédé selon l'une ou plusieurs des revendications 1 à 10, caractérisé en ce que la pression d'hydrogène est de 1 à 20 bar.

**12.** Procédé selon l'une ou plusieurs des revendications 1 à 11, caractérisé en ce que le catalyseur est choisi dans le groupe du palladium, du ruthénium, de l'iridium et du platine ainsi que de leurs complexes et sels complexes avec des ligands non-organiques ou organiques.

**13.** Procédé selon l'une ou plusieurs des revendications 1 à 12, caractérisé en ce que le catalyseur est un sel divalent du ruthénium ou un sel trivalent de l'iridium.

**14.** Procédé selon l'une ou plusieurs des revendications 1 à 13, caractérisé en ce que le catalyseur est $RuCl_2(PPh_3)_3$.

**15.** Procédé selon l'une ou plusieurs des revendications 1 à 14, caractérisé en ce que les composés de ladite formule II sont préparés à partir de dérivés de l'acide aspartique de formule V :

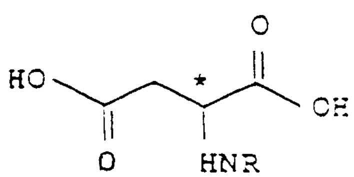

V

dans laquelle R est défini comme dans la formule (I), par l'intermédiaire de la formation d'un cycle oxazolidine, de la formation subséquente d'un chlorure d'acide et de la réduction en l'aldéhyde.

**16.** Utilisation des composés de formule II :

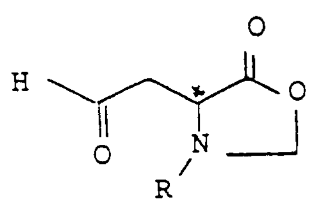

II

dans laquelle R a la même signification que dans la revendication 1, 2 ou 3, pour la préparation de lactones d'homosérine.